# EUROPÄISCHE PATENTSCHRIFT

(11) **EP 2 931 183 B1**
(45) Veröffentlichungstag und Bekanntmachung des Hinweises auf die Patenterteilung: **16.05.2018**
(21) Anmeldenummer: 13815450.5
(22) Anmeldetag: 16.12.2013
(51) Int. Cl.: A61F 2/44, A61F 2/46, A61F 2/28, A61F 2/30

(54) **STUFIG EXPANDIERBARER WIRBELZWISCHENRAUM-CAGE**
STEPWISE EXPANDABLE INTERVERTEBRAL CAGE
CAGE INTERVERTÉBRALE EXPANSIBLE GRADUELLEMENT

(30) Priorität: 14.12.2012 DE 202012011958 U; 17.12.2012 EP 12197586
(43) Veröffentlichungstag der Anmeldung: 21.10.2015
(73) Patentinhaber: FACET-LINK Inc., Rockaway NJ 07866 (US)
(72) Erfinder: DMUSCHEWSKY, Klaus, 22397 Hamburg (DE)
(74) Vertreter: Glawe, Delfs, Moll
(86) Internationale Anmeldenummer: PCT/EP2013/076697
(87) Internationale Veröffentlichungsnummer: WO 2014/091030

(56) Entgegenhaltungen:
- WO-A1-00/35389
- WO-A1-00/78253
- WO-A1-01/66047
- WO-A2-97/06753
- WO-A2-03/032812
- DE-C1- 4 012 622
- JP-A- 2000 210 315
- US-A- 5 865 848
- US-A1- 2004 162 618
- US-A1- 2007 123 987
- US-A1- 2007 255 410
- US-A1- 2008 114 467

## Beschreibung

Die Erfindung betrifft einen stufig expandierbaren Wirbelzwischenraum-Cage mit einem Boden- und einem Deckstück zur Anlage an Endplatten jeweils eines oberen und unteren benachbarten Wirbelkörpers.
Durch Verschleiß oder krankhafte Veränderungen kommt es an der Wirbelsäule zu einer Degeneration von Bandscheiben. Soweit konservative Therapie mit Indikation und/oder Physiotherapie nicht greift, ist mitunter eine operative Behandlung indiziert. Dazu ist es bekannt, in den Wirbelzwischenraum mit der degenerierten Bandscheibe ein bewegliches oder unbewegliches Implantat einzuführen. Diese übernehmen die Tragfunktion der degenerierten Bandscheibe und stellen insoweit wieder eine sichere Abstützung zwischen den benachbarten Wirbeln her. Die unbeweglichen Implantate werden auch als "Cage" bezeichnet.
Zur Implantation der Cages sind verschiedene Operationstechniken bekannt. Eine klassische Operationstechnik besteht in einem Zugang von ventral, um so die Gefahr einer Beschädigung des Rückenmarks in der Wirbelsäule zu vermeiden. Dieser Vorteil wird jedoch erkauft mit einem ausgesprochen langen Zugangsweg durch den Bauch- oder Brustraum des Patienten. Da es hierbei zu Komplikationen kommen kann, ist ein alternativer Zugangsweg etabliert worden, nämlich von dorsal. Dieser bietet zwar den Vorteil eines kurzen Wegs, jedoch besteht die Gefahr einer Kollision bzw. Verletzung des Rückenmarks. Um diese Gefahr klein zu halten, erfolgt die Operation üblicherweise im Weg minimal invasiver Chirurgie. Derartige Zugänge von unmittelbar dorsal bzw. mehr von der Seite sind als Operationstechnik PLIF (posterior lumbar intervertebral fusion) bzw. TLIF (transforaminelle interkorporelle lumbare Fusion) bekannt, bei der die Bandscheibe von posterior bzw. lateral exponiert wird. Wegen der kleinen Querschnitte beim Zugang mittels minimal invasiver Chirurgie ist hierbei die Größe des Cages naturgemäß stark beschränkt.
Für die Therapie mittels der PLIF- bzw. TLIF-Technik sind sehr kleine Cages bekannt. Sie bieten den Vorteil, dass sie durch die minimal invasive Chirurgie dank ihrer Kleinheit implantiert werden können. Ein inhärenter Nachteil ihrer Kleinheit liegt jedoch darin, dass die Stützfunktion aufgrund der geringen Abmessungen eingeschränkt und mitunter unzureichend ist. Eine größere Ausführung der Cages würde die Stützfunktion zwar verbessern, jedoch ist dies nicht praktikabel aufgrund der Beschränkung der minimal invasiven Chirurgie. Die Patentschrift WO 03/032812 A2 offenbart ein Zwischenwirbelfusionsimplantat zur Fusion zweier benachbarter Wirbel. Es umfasst ein Basisstück sowie ein Deckstück, die jeweils zur Anlage an eine zugewandte Endplatte eines der benachbarten Wirbel ausgebildet sind. Zur Höhenverstellung des Deckstücks gegenüber dem Basisstück ist eine Ratscheneinrichtung vorgesehen. Sie bewirkt eine stufige Höhenverstellung. Ferner sichert sie eine eingestellte Höhe gegenüber einem Rücklauf.

Die Erfindung hat sich zur Aufgabe gesetzt, einen Cage der eingangs genannten Art dahingehend zu verbessern, dass bei weiterhin kleinem Zugangsquerschnitt, wie er für die minimal invasive Chirurgie üblich ist, dennoch eine bessere Stützwirkung erzielen kann.
Die erfindungsgemäße Lösung liegt in den Merkmalen der unabhängigen Ansprüche. Vorteilhafte Weiterbildungen sind Gegenstand der abhängigen Ansprüche.

Bei einem Zwischenwirbelfusionsimplantat zur Fusion zweier benachbarter Wirbel umfassend ein Basisstück und ein Deckstück zur Anlage an Endplatten eines unteren und oberen der benachbarten Wirbel, wobei das Deckstück höhenverstellbar zum Basisstück ist, ist vorgesehen eine Ratscheneinrichtung zwischen Basisstück und Deckstück, die eine stufige Höhenverstellung bewirkt und eine eingestellte Höhe gegenüber einem Rücklauf sichert. Das Zwischenwirbelfusionsimplantat ferner umfasst erfindungsgemäß eine Fangeinrichtung, welche eine Verstellbewegung des Deckstücks begrenzt, und eine Auslöseeinrichtung für die Ratscheneinrichtung, um die Höhenverstellung wieder zurückzustellen. Vorzugsweise ist die Ratscheneinrichtung so ausgeführt, dass das Basisstück eine Bodenwand zur Anlage an den unteren Wirbel und eine davon aufragende Stehwand umfasst, und das Deckstück eine Deckwand zur Anlage an den oberen Wirbel und eine davon abragende Seitenwand, wobei die Stehwand und die Seitenwand Teile der Ratscheneinrichtung sind. Hierbei weist zweckmäßigerweise die Ratscheneinrichtung eine Mehrzahl von Rastnasen an der Seitenwand und eine Rastfeder an der Stehwand auf, oder umgekehrt.
Die Erfindung beruht auf dem Gedanken, durch ein höhenverstellbares Fusionsimplantat dieses bei der Implantation im kompakten Zustand durch den minimal invasiven Zugang in den Zwischenwirbelraum einzubringen, und es dort in der Höhe zu expandieren. Dies geschieht durch Betätigen eines Höhenverstellmechanismus, der als eine Ratscheneinrichtung ausgeführt ist. Damit kann eine definierte Expansion erreicht werden, die abhängt von der Anzahl der Betätigungen. Dem Chirurgen wird damit eine präzise Kontrolle über den Expansionszustand ermöglicht, und zwar auf eine rein haptische Weise. Damit ist die Erfindung frei von Beschränkungen, wie sie sich bei einer rein visuellen Kontrolle des Expansionszustands (ggf. unter Röntgenkontrolle) ergeben würden.

Weiter wird mit der Ratscheneinrichtung eine Verriegelung des einmal eingestellten Expansionszustands erreicht. Dank des Formschlusses der Ratscheneinrichtung ist damit ein Schutz vor unbeabsichtigter Rückstellung auch bei sehr hohen Lastkräften erreicht. Damit ergibt sich eine hervorragende Stabilität, und zwar auch und gerade unmittelbar nach der Operation, bis durch Knochenwachstum schließlich eine vollständige Fusion der beiden benachbarten Wirbelkörper erreicht ist.

Mit Vorteil ist eine doppelte Ratscheneinrichtung vorgesehen, und zwar vorzugsweise eine an jeder Lateralseite des Implantats. Dies ergibt zum einen eine sicherere Abstützung auch bei nicht zentrisch auftretenden Lasten. Zum anderen ermöglicht es eine solche doppelte Ratscheneinrichtung, unterschiedliche Höhen für die beiden Seiten einzustellen, so dass es zu einer definierten Verkippung des Deckstücks zum Basisstück kommt. Damit kann eine Anpassung an Verkrümmungen in der Wirbelsäule in dem jeweiligen Zwischenwirbelraum vorgenommen werden. Dazu kann auch vorgesehen sein, dass die beiden Ratscheneinrichtungen höhenmäßig versetzt zueinander sind, und zwar vorzugsweise durch einen Versatz ihrer jeweiligen Rastnasen.

Bevorzugt sind zwei Stehwände seitlich an der Bodenwand vorgesehen, und die Bodenwand ist elastisch. Dies ermöglicht ein flexibles, reversibles Aufspreizen der beiden Stehwände, so dass die Expansionsbewegung vereinfacht wird. Gleichzeitig kann durch planvolles Aufspreizen der beiden Stehwände auf Wunsch eine Rückstellmöglichkeit für das Deckstück geschaffen werden. Dies ermöglicht ein Testen des Fusionsimplantats bzw. ein Rücksetzen bei einer Überexpansion. Bei einer alternativen Variante sind ausschließlich die Stehwände elastisch ausgeführt, während die Bodenwand steif ist. Damit wird eine Wölbung der Bodenwand vermieden. Vorzugsweise ist das Deckstück aus einem weniger elastischen Material gefertigt als das Bodenstück. Beispielsweise besteht das Deckstück aus einer Kobalt-Chrom-Legierung, während das Basisstück aus einer elastischeren Titanlegierung besteht. Damit kann eine größere Kraftaufnahmefähigkeit erreicht werden, insbesondere an den Rastnasen der Ratscheneinrichtung.
Um eine unbeabsichtigte Überexpansion zu verhindern, ist eine Fangeinrichtung vorgesehen, welche die Verstellbewegung des Deckstücks begrenzt. Zweckmäßigerweise ist die Fangeinrichtung durch eine Sperrklinke gebildet, die mit einem Schultervorsprung zusammenwirkt. Damit wird auf einfache und effiziente Weise eine schädliche Überexpansion verhindert. Bei einer alternativen Ausführungsform ist die Fangeinrichtung von Fenstern in den Stehwänden und einem seitlich aus den Seitenwänden ragenden, in die Fenster eingreifenden Querstift gebildet. Vorzugsweise ist der Querstift unten an den Seitenwänden angeordnet. Damit können die Rastzähne an den Seitenwänden ununterbrochen durchlaufen, was die Kraftübertragung verbessert.

Um eine Feineinstellung zu ermöglichen, sind bei doppelten Ratscheneinrichtungen die Rastnasen an einer der beiden Ratscheneinrichtungen vorzugsweise so ausgeführt, dass sie einen Abstand aufweisen, der ein ganzzahliges Vielfaches beträgt des Abstands der Rastnasen an der anderen der beiden Ratscheneinrichtungen. Beispielsweise ist der Abstand an der einen Ratscheneinrichtung doppelt so groß wie der an der anderen. Damit kann einerseits eine Schnellverstellung durch den größeren Abstand erreicht werden. Andererseits kann, und das ist in der Praxis ein erheblicher Vorteil, unter Nutzung der Ratscheineinrichtung mit dem kleineren Abstand eine feinere Einstellung erreicht werden, und zwar insbesondere im Hinblick auf die Einstellung eines Verkippungswinkels zwischen Deckstück und Basisstück.

Ein bewährtes Maß für den Abstand der Rastnasen voneinander beträgt etwa das 1,5 bis 3,0-fache ihrer Erhebung (die definiert ist durch den Abstand zwischen Zahnspitze und Zahngrund).

Die Rastnasen sind mit Vorteil asymmetrisch, vorzugsweise sägezahnförmig, ausgebildet. Das erlaubt eine Gestaltung derart, dass bei der Expansion dank günstiger Flankenwinkel nur eine geringe Kraft erforderlich ist, während gegenüber einer Rückstellung hohe Haltekräfte aufgebracht werden. Bewährt hat es sich, wenn der Winkel einer tragenden Flanke im Bereich von 5 bis 35° liegt, vorzugsweise bei etwa 10°. Unter der tragenden Flanke wird diejenige verstanden, welche die Last trägt und somit einer Rückstellung entgegenwirkt. Die Sägezahnform ist vorzugsweise so gestaltet, dass sich ein Zahnwinkel im Bereich von 45° bis 70°, vorzugsweise von etwa 60° ergibt. Bei einer besonders zweckmäßigen Ausführungsform überlappen sich die Rastnasen einander schuppig, wobei die Gegenflanke vorzugsweise gleichsinnig, aber flacher als die tragende Flanke ausgerichtet ist. Diese Gestaltung ermöglicht eine besonders günstige Einstellung der Kraftverhältnisse zwischen Betätigungskraft bei der Expansion und Haltekraft gegenüber Rückstellung. Dies kann noch gesteigert werden, wenn die Rastfeder formschlüssig in die Rastnasen eingreift, wobei vorzugsweise die Form der Rastnasen so auf die Rastfeder abgestimmt ist, dass eine Selbstsicherung erreicht ist. Damit ergibt sich eine maximale Sicherung gegenüber unerwünschter Rückstellung, die letztlich nur noch durch die Belastungsgrenzen des Materials gegenüber Bruch oder Abscherung bestimmt sind.

Zur Maximierung der Selbstsicherung sind die miteinander zusammenwirkenden Flächen an Rastnase und Rastfeder nicht parallel, sondern spitzwinklig zueinander orientiert. Unter spitzwinklig wird hierbei verstanden, dass die jeweiligen Flächen in Richtung zum Grund der Rastzähne voneinander divergieren. Mit dieser Gestaltung wird erreicht, dass sich unter Last die Rastfeder weiter zwischen die Rastzähne zieht. Ein unerwünschtes Herausspringen der Rastfeder aus den Rastzähnen wird damit sicher vermieden. Die Sicherungswirkung wird so maximiert.

Vorzugsweise ist zwischen Deckstück und Basisstück ein innerer Hohlraum gebildet. Dies ermöglicht ein Einbringen von Knochenmaterial, wie Grafts oder Chips, um so ein Einwachsen von Knochenmaterial zur Versteifung zu begünstigen. Um ein Einbringen des Knochenmaterials und insbesondere auch das Einwachsen von den beiden benachbarten Wirbeln her zu begünstigen, ist vorzugsweise an der Deckplatte und/oder Bodenwand mindestens eine Durchbrechung vorgesehen, die mit dem Hohlraum verbunden ist.

Weiter kann ein Sperrelement vorgesehen sein, welches in den Hohlraum einschiebbar ist. Damit kann nach Implantation und Expandieren auf die gewünschte Höhe das Deckstück in seiner Position fixiert werden. Es ergibt sich so eine zusätzliche Sicherungswirkung. Vorzugsweise sind mehrere Sperrelemente mit verschiedenen Höhen vorgesehen, so dass je nach Expansion des Fusionsimplantats ein passendes Sperrelement eingeschoben werden kann. Vorzugsweise ist am Sperrelement mindestens eine Zusatzdurchbrechung vorgesehen, die mit der Durchbrechung an der Deckplatte und/oder Bodenwand fluchtet. Somit kann auch bei eingeschobenem Sperrelement das Einwachsen von Knochenmaterial in den Hohlraum begünstigt werden. Weiter vorzugsweise können das Deckstück und/oder Basisstück an seinen Stirnseiten Öffnungen aufweisen. Damit wird einerseits das Einwachsen weiter gefördert, und andererseits kann auch noch nach Implantation Knochenmaterial durch diese stirnseitige Öffnung in den Hohlraum eingebracht werden.

Mit Vorteil ist das Deckstück und/oder Basisstück an seinen Stirnseiten abgerundet, insbesondere an seinen Ecken. Dies erleichtert das Einschieben des erfindungsgemäßen Fusionsimplantats in den Zwischenwirbelraum.

Vorzugsweise ist die Oberseite des Deckstücks und/oder die Unterseite des Basisstücks strukturiert ausgeführt. Darunter wird verstanden, dass sie regelmäßige Erhebungen oder Vertiefungen aufweist. Bevorzugt sind längs, also in Richtung von Stirnfläche zu Stirnfläche, verlaufende feine Rillen und/oder quer, also von Seitenwand zu Seitenwand, verlaufende Auskehlungen. Die Rillen weisen einen Abstand von mindestens 0,2 mm bis höchstens 1 mm auf, während die Auskehlungen eine Weite von mindestens 2 mm und vorzugsweise nicht mehr als 5 mm aufweisen. Die feinen Rillen wirken wie eine Zahnung und halten das Zwischenwirbelfusionsimplantat an seinem Platz. Die Auskehlungen schützen es vor einem Auswandern insbesondere unter Kraftbeaufschlagung, wie sie beim Bücken oder Beugen des Patienten häufig vorkommt. Außerdem sichern sie initial das Zwischenwirbelfusionsimplantat vor einem Herausrutschen. Mit Vorteil sind die Oberseite und/oder Unterseite mit einer Beschichtung aus Titanplasmaspray versehen, welches ein Einwachsen von Knochenmaterial begünstigt. Das Titanplasmaspray ist vorzugsweise feinkörnig mit einer Körnung von höchstens 90 µm, so dass es auch feine Strukturen wie die besagten Rillen nicht verschließt. Bevorzugterweise sind hierbei die randnahen Bereiche zu den Stirnseiten hin glatt oder nur mit den Rillen versehen, nicht aber mit den Auskehlungen. Damit wird eine größere Lasttragefähigkeit für den kortikalen Wirbelrand sichergestellt.

Die Gesamtgestaltung des Zwischenwirbelfusionsimplantats ist vorzugsweise generell kastenförmig. Dies ist fertigungstechnisch günstig und vereinfacht ein gerades Einbringen des Implantats in den Zwischenwirbelraum. Es kann aber auch gewünscht sein, dass die Implantation nicht gerade, sondern vielmehr bogenförmig erfolgt. Dazu ist vorzugsweise das Zwischenwirbelfusionsimplantat kreisbogenartig gekrümmt ausgebildet. Dies vereinfacht eine Implantation insbesondere bei einem Zugang von schräg hinten mittels des sog. TLIF-Verfahrens (transforaminelle interkorporelle lumbare Fusion). Ferner kann das Zwischenwirbelfusionsimplantat keilförmig sein in der Weise, dass eine Stirnwand niedriger ist als die andere. Bevorzugt sind Keilwinkel im Bereich von etwa 3° bis 15°. Damit kann eine bessere Anpassung an eine Krümmung der Wirbelsäule erfolgen, insbesondere an eine Lordose.

Es kann zweckmäßig sein, das bereits expandierte Zwischenwirbelfusionsimplantat zurückzustellen auf das kompakte Maß, wie es für die Implantation eingestellt ist. Dies kann nach einem extrakorporalen Test auf Funktionstüchtigkeit oder im Anschluss an eine ggf. testweise erfolgte (Über-)Expansion erfolgen. Um das Zwischenwirbelfusionsimplantat wieder zurückstellen zu können, ist eine Auslöseeinrichtung für die Ratscheneinrichtung vorgesehen. Sie kann in einem einfachen Fall realisiert sein mittels am freien Ende der Stehwand und/oder Seitenwand angeordneter Aufnahmen für ein lateral wirkendes Spreizelement. Dieses spreizt die Stehwände auf, so dass die Rastzähne sich aus dem Eingriffsbereich der Rastfeder bewegen, und das Deckstück kann relativ zu dem Basisstück wieder zurückgestellt werden. Bei einer bevorzugten Ausführungsform sind Aufnahmen als Löcher am oberen Ende der Stehwand ausgeführt, vorzugsweise im Bereich der Sperrklinke. Dies ermöglicht eine besonders einfache Rückstellungsoption, wenn auch nur in einem Zustand außerhalb des Wirbelzwischenraums.

Als Spreizelement eignet sich besonders ein zangenartiges Instrument, welches mit seinen Greifern in die Aufnahmen fasst.
Die Erfindung erstreckt sich weiter auf eine Anordnung aus einem Zwischenwirbelfusionsimplantat gemäß der in den Ansprüchen definierten Erfindung und einem Betätigungsinstrument, welches zum Betätigen der Höhenverstellung ausgebildet ist.
Erfindungsgemäß ist hierbei das Betätigungsinstrument so gestaltet, dass es einen Querschnitt aufweist, der höchstens so groß wie der des Zwischenwirbelfusionsimplantats ist, das Betätigungsinstrument eine Anzeigeeinrichtung aufweist, welche eine Darstellung proportional zur Höhenverstellung des Zwischenwirbelfusionsimplantats angibt. Damit ist sichergestellt, dass die Expansion unter Nutzung des Betätigungsinstruments durch denselben kleinen minimal invasiven Zugangsweg erfolgen kann wie das eigentliche Einsetzen des Zwischenwirbelfusionsimplantats. Ein zweiter gesonderter Zugangsweg ist damit nicht erforderlich. Es ergibt sich somit sowohl eine Aufwandsverringerung wie auch eine Patientenschonung.

Das Betätigungsinstrument kann als ein Ziehkeilexpander mit zwei Spreizbacken ausgeführt sein. Dabei ist der Ziehkeil vorzugsweise im vorderen Bereich an den Spreizbacken angeordnet. Durch Bewegen des Ziehkeils kann eine Expansion der Spreizbacken bewirkt werden, welche wiederum von innen auf das Deckstück bzw. das Basisstück wirken und diese somit expandieren.
Vorzugsweise ist für die Bewegung des Ziehkeils ein Zugorgan vorgesehen, dass zwischen den Spreizbacken nach hinten geführt ist. Unter hinten wird hier eine Führung weg vom Implantat nach außen in Richtung zum Chirurgen verstanden. Dies ermöglicht dem Chirurgen eine bequeme Betätigung von außen durch den minimal invasiven Zugang. Die Führung des Zugorgans zwischen den Spreizbacken ermöglicht einen kompakten Aufbau. Weiterhin ermöglicht dies einen guten Schutz des Zugorgans vor mechanischer Beeinträchtigung. Vorteilhaft ist eine Ausführung des Zugorgans als eine Betätigungsspindel. Sie ermöglicht eine präzise Verstellung und erlaubt außerdem eine reversible Betätigung durch Verdrehen in Gegenrichtung.
Bei einer bewährten Ausführungsform ist am vorderen Ende der Betätigungsspindel der Ziehkeil angeordnet, und am hinteren außenliegenden Ende ist ein Verstellrad gelagert. Dies ermöglicht eine bequeme reversible Betätigung von außen.
Grundsätzlich kann es ausreichen, wenn durch eine taktile Rückmeldung der Chirurg eine Vorstellung über den Umfang der Höhenverstellung erhält. Es ist aber von Vorteil, wenn das Betätigungsinstrument eine Anzeigeeinrichtung aufweist, welche die erreichte Höhenverstellung des Zwischenwirbelfusionsimplantats angibt. Die Anzeigeeinrichtung weist dazu eine Darstellung proportional zur erreichten Höhenverstellung auf. Dem Chirurgen wird damit eine Bestimmung der bereits erreichten Expansion erleichtert.
Alternativ kann ein Betätigungsinstrument auch als ein Drehhebelsteller vorgesehen sein. Es umfasst einen Führteil und einen darin schwenkbeweglich geführten Hebelteil, wobei an beiden Aufnahmen für entweder das Basisstück oder das Deckstück vorgesehen sind. Zweckmäßigerweise ist es so gestaltet, dass das Basisstück an einem vorderen Ende des Führteils gehaltert ist, während das Deckstück drehfest an dem Hebelteil gehaltert ist. Hierfür ist zweckmäßigerweise eine Schraubverbindung am vorderen Ende des Hebelteils vorgesehen, welche in ein entsprechendes Gegengewinde einer Öffnung in der Stirnseite des Deckstücks greift.
Das Hebelteil weist vorzugsweise einen Knebel zur Betätigung an seinem hinteren Ende auf. Es ist gelagert in dem Führteil, das dazu so ausgebildet ist, dass das Hebelteil in Radialrichtung beweglich gelagert ist. Das bedeutet,
dass durch Verschwenken des Knebels in Radialrichtung das Hebelteil sich entsprechend dreht.
Das Hebelteil weist vorzugsweise weiter eine Axialbohrung auf, durch welche eine Befestigungsspindel gesteckt ist. Sie dient zur leicht lösbaren Befestigung des Deckstücks an dem Hebelteil. Bewährt hat es sich, an dem Führteil eine Glocke mit einer Ausnehmung für den Knebel vorzusehen. Hierbei ist die Ausnehmung so gestaltet, dass sie in Radialrichtung eine größere Weite aufweist, als es einer Dicke des Knebels entspricht. Damit ist der Knebel in Radialrichtung beweglich in der Glocke und kann somit hin- und herbewegt werden. Diese Hin- und Herbewegung überträgt sich auf das an dem vorderen Ende festgeschraubte Deckstück, welches sich entsprechend verkippt und damit wechselweise an den beiden Ratscheneinrichtungen je einen Rastzahn weiter nach oben wandert. Auf diese Weise wird eine Höhenverstellung bewirkt. Ferner kann, sofern eine Verkippung gewünscht ist, durch eine ungerade Anzahl von Betätigungsvorgängen eine Verkippung eingestellt werden.

Ein weiteres alternatives Betätigungsinstrument ist als ein Parallelspreizer mit Kreuzhebel ausgeführt. Der Kreuzhebel bildet eine Umkehrkinematik, wodurch sich der Spreizer bei einem Druck auf den Griff spreizt. Diese Kinematik ist günstiger in der Anwendung als eine einfache Zange ohne Kreuzhebel, wo der Griff gespreizt werden müsste. Ferner weist der Parallelspreizer vorzugsweise eine verstellbare Anschlageinrichtung auf. Mit ihr kann eine Höchstspreizung festgelegt sein, die dann nicht überschritten wird. Das ist insbesondere bei Implantationen mit schwierigen Zugangsverhältnissen von Vorteil, da so der Chirurg während des Spreizens nicht mehr die erreichte Stellung manuell kontrollieren muss. Zweckmäßigerweise ist ein Drehrad mit mehreren vordefinierten und beschrifteten Positionen vorgesehen. Damit wird die Anschlageinrichtung entsprechend verstellt. So kann der gewünschte Spreizgrad einfach eingestellt werden.

Vorzugsweise sind die Spreizbacken verschieden im Hinblick auf die Halterung von Basis- bzw. Deckstück ausgeführt. Vorzugsweise sind nur an einer der Spreizbacken Einrichtungen zur formschlüssigen Aufnahme angeordnet, und zwar vorzugsweise als in Durchbrechungen im Basisstück greifende Vorsprünge. Durch die verschiedene Gestaltung wird eine fehlersichere Kodierung erreicht in dem Sinne, dass das Zwischenwirbelfusionsimplantat nur mit einer Orientierung richtig von dem Betätigungsinstrument aufgenommen werden kann. Fehler durch eine verkehrt herum erfolgende Aufnahme sind somit ausgeschlossen.

Die Erfindung wird nachfolgend unter Bezugnahme auf die beigefügte Zeichnung anhand vorteilhafter Ausführungsbeispiele näher erläutert. Es zeigen:
- Fig. 1:: eine Ansicht eines Ausführungsbeispiels für ein Zwischenwirbelfusionsimplantat im implantierten Zustand an Wirbelkörpern;
- Fig. 2:: perspektivische Ansichten von Basisstück und Deckstück gemäß der in Fig. 1 dargstellten Ausführungsform;
- Fig. 3:: schematische Darstellungen zur Wirkung einer Ratscheneinrichtung;
- Fig. 4:: eine vergrößerte Darstellung von Zähnen der Ratscheneinrichtung gemäß Fig. 3;
- Fig. 5:: ein Beispiel für eine Fangeinrichtung bei einer Variante des Ausführungsbeispiels;
- Fig. 6:: Darstellungen zu einer Auslöseeinrichtung in verriegelter und entriegelter Stellung;
- Fig. 7:: eine perspektivische sowie eine Schnittdarstellung zu einem Sicherungselement im demontierten und montierten Zustand;
- Fig. 8:: eine perspektivische Darstellung eines Greifkopfes für ein Betätigungsinstrument;
- Fig. 9:: Schnittdarstellungen zu dem Betätigungsinstrument gemäß Fig. 8 in entspanntem und teilweise expandiertem Zustand;
- Fig. 10:: eine Aufsicht sowie Detaildarstellungen im Schnitt zu dem Betätigungsinstrument;
- Fig. 11:: eine perspektivische Ansicht einer zweiten Ausführungsform;
- Fig. 12:: ein zweiteiliges Betätigungsinstrument für eine Zwischenwirbelraumfusionsprothese gemäß einer dritten Ausführungsform;
- Fig. 13:: Darstellungen zu dem zweiten Betätigungsinstrument in teilweise montiertem und vollständig montiertem Zustand;
- Fig. 14:: eine perspektivische Darstellung eines weitern Ausführungsbeispiels für das Zwischenwirbelfusionsimplantat;
- Fig. 15:: eine Schnittansicht zu Fig. 14;
- Fig. 16:: eine Detailvergrößerung zu Fig. 14;
- Fig. 17:: eine Seitenansicht einer Variante zu Fig. 14;
- Fig. 18:: eine Aufsicht auf eine vierte Ausführungsform des Betätigungsinstruments;
- Fig. 19:: eine Detailvergrößerung zu Fig. 18; und
- Fig. 20:: eine weitere Detailvergrößerung zu Fig. 18.

Ein in seiner Gesamtheit mit der Bezugsziffer 1 bezeichnetes Zwischenwirbelfusionsimplantat ist zur Implantation in einem Wirbelzwischenraum 91 zwischen zwei unmittelbar benachbarten Wirbelkörpern 9, 9' vorgesehen. Bei physiologisch intakter Wirbelsäule befindet sich zwischen den Wirbeln im Wirbelzwischenraum eine Bandscheibe 90. Sie kann aufgrund von Krankheit oder Verschleiß degeneriert sein, so dass sie zumindest teilweise reseziert werden muss. Um trotz des Verlustes an Bandscheibenmaterial eine ausreichende Abstützung des Wirbelzwischenraums 91 zu erreichen und somit ein Kollabieren der Wirbelsäule zu verhindern, ist das Zwischenwirbelfusionsimplantat 1 in den Wirbelzwischenraum 91 eingesetzt. Es wirkt stützend und erleichtert somit eine Fusion der beiden benachbarten Wirbel 9, 9' auf natürlichem Wege durch Knochenwuchs.

Das Zwischenwirbelfusionsimplantat 1 umfasst ein Basisstück 2, welches auf einer oberen Endplatte 92 des unteren benachbarten Wirbels 9 aufliegt. Ferner umfasst es ein Deckstück 3, welches an einer unteren Endplatte 93 des oberen benachbarten Wirbels 9' anliegt. Um eine sichere Anlage des Basisstücks 2 an der unteren Endplatte 92 einerseits und des Deckstücks 3 an der unteren Endplatte 93 des oberen Wirbels 9' andererseits zu gewährleisten, ist es erforderlich, das Zwischenwirbelfusionsimplantat 1 in seiner Höhe entsprechend einzustellen. Es muss so weit expandiert werden, bis das Basisstück 2 und das Deckstück 3 an den jeweiligen Endplatten 92, 93 fest anliegen.

Um ein Auseinanderbewegen von Basisstück 2 und Deckstück 3 im Sinne einer Expansion zu ermöglichen, ist eine Ratscheneinrichtung 4 vorgesehen. Sie umfasst eine Vielzahl von Rastzähnen 43 an dem Deckstück, welche mit einer Rastfeder 42 am Basisstück 2 zusammenwirken.

Um einerseits das erfindungsgemäße Zwischenwirbelfusionsimplantat 1 kompakt zu halten und so durch einen im Wege der minimal invasiven Chirurgie bereiteten Zugang implantieren zu können, aber andererseits eine ausreichend große Stützwirkung im Wirbelzwischenraum 91 entfalten zu können, ist das erfindungsgemäße Zwischenwirbelfusionsimplantat 1 höhenverstellbar ausgeführt. Dazu weist es ein Basisstück 2 und ein Deckstück 3 auf, das in seiner Höhe beweglich relativ zu dem Basisstück 2 angeordnet ist. Das Basisstück 2 ist im Wesentlichen trogförmig ausgeführt und umfasst eine Bodenwand 22, welche als Anlagefläche an die Endplatte 92 des unteren der benachbarten Wirbel 9 fungiert, sowie an beiden Lateralseiten je eine senkrecht nach oben ragende Stehwand 21. Das Basisstück 2 ist somit im Querschnitt insgesamt U-förmig. Am oberen Ende der Stehwand 21 ist die Rastfeder 42 als ein Teil der Ratscheneinrichtung 4 angeordnet. Ferner ist am oberen Ende der Stehwand 21 etwa mittig ein Vorsprung 52 angeordnet, welcher einen Teil einer Fangeinrichtung 5 bildet. In der Bodenwand 22 sind mehrere Öffnungen 24 ausgeformt, welche einen Hohlraum 10 im Inneren der trogförmigen Gestaltung zwischen den Seitenwänden 21 verbinden mit dem Raum unterhalb der Bodenwand 22. Dies begünstigt das Einwachsen von Knochen in das Zwischenwirbelfusionsimplantat 1.

Weiter umfasst das dargestellte Ausführungsbeispiel für das erfindungsgemäße Zwischenwirbelfusionsimplantat 1 ein Deckstück 3. Es weist eine generell kastenartige Gestalt auf mit einer im Wesentlichen planen Oberseite 33, welche als Anlagefläche für die untere Endplatte 93 des oberen benachbarten Wirbels 9' fungiert. An den Lateralseiten sind je eine Seitenwand 31 nach unten abragend angeordnet. Sie tragen an ihrer nach außen weisenden Fläche eine Mehrzahl von Rastzähnen 43, welche einen Teil der Ratscheneinrichtung 4 bilden und mit der Rastfeder 42 am Basisstück 2 zusammenwirken. Bei dem dargestellten Ausführungsbeispiel ist an einer Stirnseite eine Stirnwand 30 vorgesehen. Sie ist optional und kann auch entfallen. Weiter ist an den Seitenwänden 31 etwa in der Mitte eine Anlageschulter 53 ausgeformt. Sie ist von ihren Abmessungen so gewählt, dass sie mit dem Vorsprung 52 an dem Basisstück 2 zusammenwirkt. Hierbei ist die Anordnung der Anlageschulter 53 so gewählt, dass sie zur Anlage an den Vorsprung 52 in der maximalen Extensionsposition des Deckstücks 3 relativ zum Basisstück 2 kommt. Damit wird eine Anschlagbegrenzung erreicht. Der Vorsprung 52 und die Anlageschulter 53 wirken somit als eine Fangeinrichtung 5 zusammen. An der Oberseite 33 sind mehrere Durchbrechungen 34 ausgeformt, um Knochenmaterial ein Einwachsen in das Innere des Deckstücks zu ermöglichen.

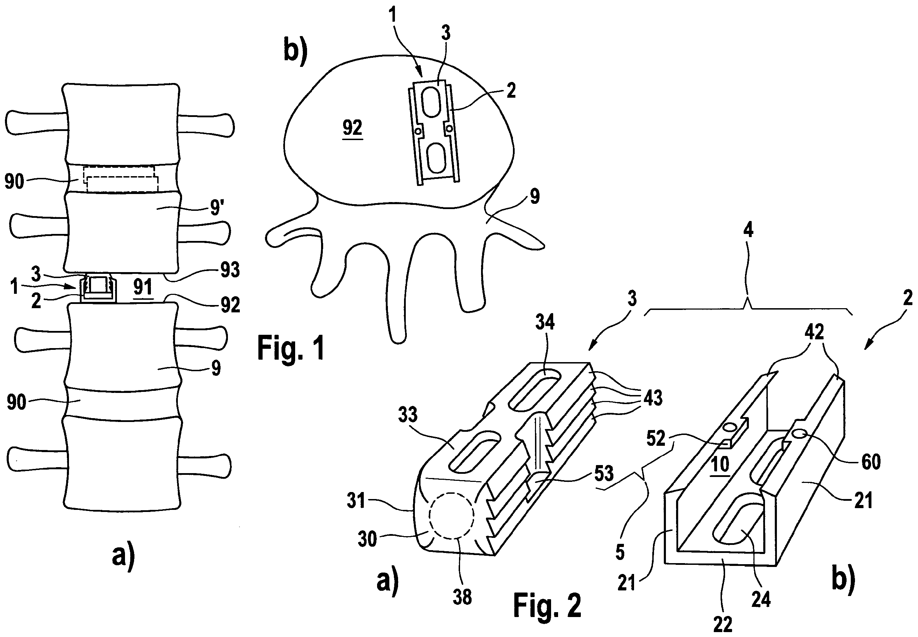

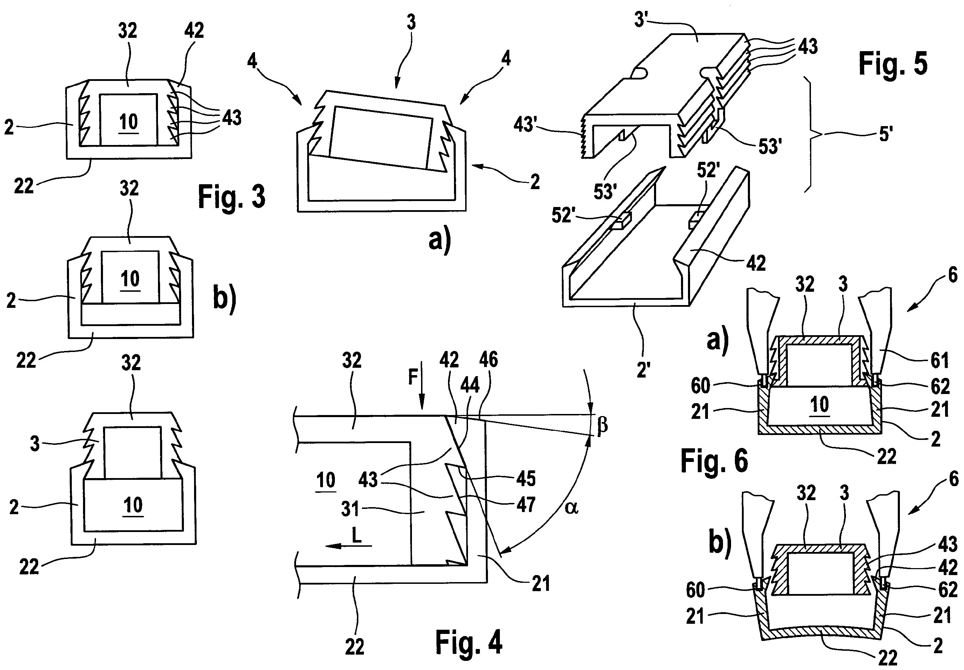

Zur Implantation wird das Deckstück 3 in den Hohlraum 10 des trogförmigen Basisstücks 2 eingesetzt. Das Implantat ist damit in dem Zustand minimaler Expansion, wie es in Fig. 3a in der obersten der drei übereinander angeordneten Darstellungen abgebildet ist. In diesem Zustand wird das Implantat in den Zwischenwirbelraum 91 eingeführt, bis es etwa die Position erreicht, welche in Fig. 1b dargestellt ist. Es kann dann in einem nächsten Schritt so weit expandiert werden, bis es eingestellt ist auf die Höhe des Zwischenwirbelraums 91. Dies geschieht in der Weise, indem das Deckstück 3 mittels eines Betätigungsinstruments 8 nach oben bewegt wird, wobei sukzessive die Rastfeder 42 nacheinander mit verschiedenen Rastzähnen 43 an den Lateralseiten 31 des Deckstücks 3 eingreift und so die erreichte Expansionsposition sichert. Dieser Vorgang der Expansion ist in den drei Darstellungen in Fig. 3a abgebildet.

Die Rastzähne 43 sind sägezahnartig geformt. Jeder Zahn 43 weist zwei Flanken 45, 47 auf. Hierbei ist die kürzere Flanke 45 die lasttragende Flanke, welche kraftübertragend in Eingriff mit der Rastfeder 42 kommt. Diese lasttragende Flanke 45 ist nach unten weisend geformt und damit in derselben Richtung orientiert wie die andere, nicht lasttragende Anlageflanke 47. Dadurch ergibt sich eine gewisse schuppige Überlappung der Rastzähne 43 bei einer Betrachtungsweise von der Seite. Bei dem in Fig. 4 dargestellten Ausführungsbeispiel sind die Winkel α und β so gewählt, dass der Winkel α etwa 60° und der Winkel β 8° beträgt. Die Oberseite 46 der Rastfeder 42 ist um den besagten Winkel β geneigt. Damit ergibt sich zusammen mit der nach unten weisenden Orientierung der lasttragenden Flanke 45 unter der Wirkung einer Lastkraft F eine nach innen gerichtete Lateralkraft L, welche die Rastfeder 42 in die Verzahnung 43 hineinzieht und diese damit gegenüber einem unerwünschten Herausspringen aus den Rastzähnen 43 sichert. Hiermit wird eine Selbstsicherung in der Weise erreicht, dass die Sicherungskraft L umso größer wird, je größer die Lastkraft F ist.

Um zu verhindern, dass bei der Expansion das Deckstück zu weit nach oben bewegt wird und damit die Rastfeder 42 aus dem Bereich der Rastzähne 43 gerät, ist alternativ eine Fangeinrichtung 5', wie in Fig. 5 dargestellt, vorgesehen. Sie umfasst einen Vorsprung 52' an jeder Rastfeder 42, und zwar an deren Mitte. An dem Deckstück 3 ist unterhalb der Rastzähne 43 ein nach unten weisender Bügel 53' angeordnet, dessen Abmessungen so gewählt sind, dass sie den Vorsprung 52' umschließen. Ist die maximale Expansion erreicht, so greift der Bügel 53' unter den Vorsprung 52' und verhindert eine weitere Expansionsbewegung.

Die Expansionsbewegung erfolgt zum Zwecke der Höhenverstellung in der Regel auf beiden Seiten der Ratscheneinrichtung 4 gleichmäßig, wie auch in Fig. 3a abgebildet. Jedoch kann es auch gewünscht sein, dass zur Einstellung eines bestimmten Kippwinkels die Verstellung ungleichmäßig erfolgt. Je nachdem, ob auf der einen Seite die Verstellung um ein oder zwei Rastzähne 43 mehr erfolgt, kann ein gewünschter Kippwinkel definiert eingestellt werden. Der Chirurg braucht dazu nur die Anzahl der "Klicks" abzuzählen, d. h. die Anzahl der Rastvorgänge in der Ratscheneinrichtung 4 durch das Eingreifen der Rastfeder 42 in die verschiedenen Rastzähne 43. Für eine feinere Einstellung der Verkippung können auch feiner gestufte Zähne 43' vorgesehen sein, doppelt so viele Zähne 43' mit jeweils der halben Größe verglichen mit den Zähnen 43. Zweckmäßigerweise ist nur eine der beiden Ratscheneinrichtungen mit den feineren Zähnen versehen, wie in Fig. 5 dargestellt ist. Optional sind die Startpunkte der Zähne 43, 43' zwischen den beiden Ratscheneinrichtungen versetzt zueinander.

Es kann erforderlich sein, das Deckstück 3 aus seiner expandierten Position wieder zurückzustellen in eine weniger expandierte oder überhaupt nicht mehr expandierte Position, wie sie zur Implantation verwendet wird. Dazu ist zweckmäßigerweise eine Rücksetzeinrichtung 6 vorgesehen. Sie umfasst ein Instrument mit zangenartigen Greifern 61, welche mit entsprechenden Greifspitzen 62 an ihrer Vorderseite in zu diesem Zweck vorgesehene Aufnahmeöffnungen 60 an der Oberseite der Stehwände 21 greifen. Durch Spreizen der Greifer 61 mit einer zangenartigen Bewegung werden die beiden gegenüberliegenden Stehwände 21 voneinander weg bewegt, wodurch die Rastfeder 42 an der jeweiligen Stehwand 21 nicht mehr im Eingriff steht mit den Rastzähnen 43 an dem Deckstück 3. Damit wird die Ratscheneinrichtung 4 freigegeben. Das Deckstück 3 kann frei nach unten bewegt werden und auf diese Weise zurückgestellt werden. Vorzugsweise ist das Material des Basisstücks 2 zu diesem Zweck so gewählt, dass es sich elastisch verformen lässt, insbesondere die Bodenwand 22. Dies ist in Fig. 6 dargestellt.

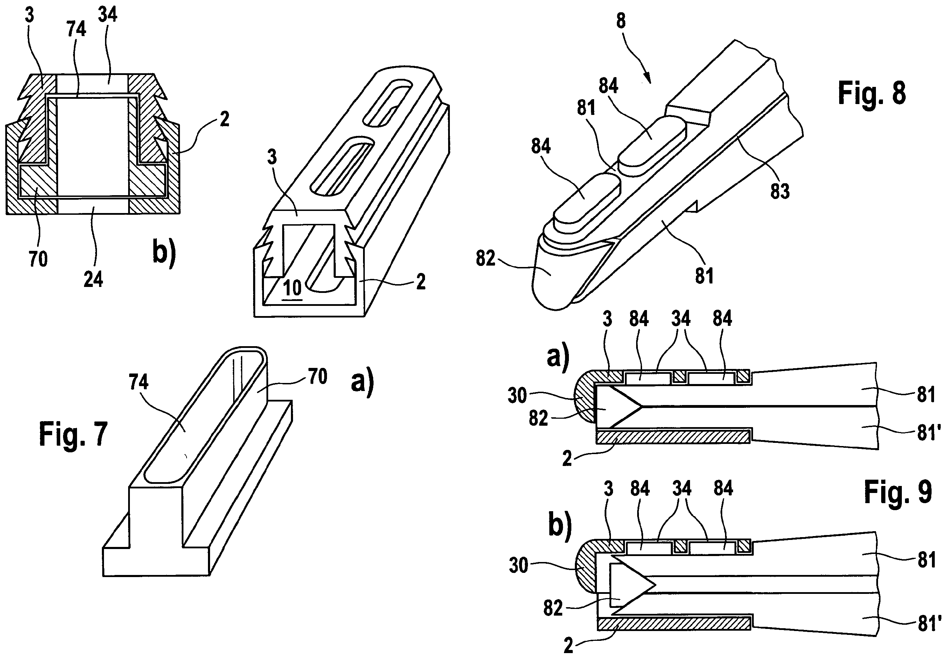

Um nach Erfolg der Expansion eine zusätzliche Sicherung für die erreichte Höheneinstellung zu erzielen, kann ein gesondertes Sperrelement 70 vorgesehen sein. Es ist vorzugsweise so geformt, dass es in den bei der Expansion vergrößerten Hohlraum 10 zwischen Deckstück 3 und Basisstück 2 passend eingeschoben werden kann. Es versteht sich, dass hierbei je nach Expansionsstatus verschiedene Sperrelemente 70 verwendet werden müssen. Das Sperrelement 70 weist vorzugsweise eine zentrale Durchbrechung 74 auf, welche mit den Durchbrechungen 34 am Deckstück 3 bzw. 24 am Basisstück 2 fluchtet. Damit ist der Hohlraum 10 weiter leicht erreichbar und zugänglich zum Einwachsen von Knochenmaterial. Das Sperrelement 70 sichert im eingeschobenen Zustand das Deckstück 3 gegenüber einem Rücklauf zurück in das Basisstück 2, wie es in Fig. 7b dargestellt ist.

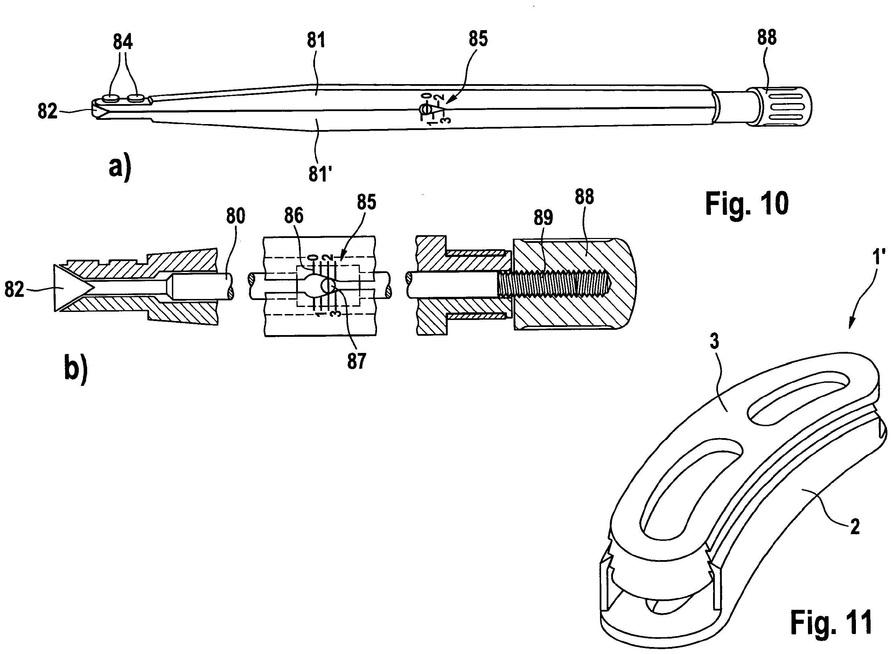

Zur Höheneinstellung mittels Expansion ist ein Betätigungsinstrument vorgesehen. Es ist als ein Ziehkeilexpander 8 ausgeführt mit zwei Spreizbacken 81. Sie sind an ihrem vorderen Ende mit Aufnahmen 84 versehen, welche formschlüssig in die Durchbrechungen 34 greifen. Am vorderen Ende sind die Spreizbacken 81 V-förmig eingeschnitten, wobei in diesem Einschnitt ein keilartig geformter Ziehkeil 82 eingesetzt ist. Der Ziehkeil 82 ist verbunden mit einem Zugorgan 80, welches sich entlang der Längsachse des Betätigungsinstruments 8 über dessen gesamte Länge erstreckt (siehe Fig. 10). Es beginnt mit seinem vorderen Ende an dem Ziehkeil 82 und endet am hinteren Ende des Betätigungsinstruments 8 in einem Handrad 88, in welchem es über ein Spindelgewinde 89 gelagert ist. Durch Verdrehen des Handrads 88 wird mittels des Spindelgewindes 89 das Zugorgan 80 nach hinten bewegt, wodurch sich der Ziehkeil 82 in die V-förmige Vertiefung zwischen den beiden Spreizbacken 81 einsenkt. Die Spreizbacken 81 werden dadurch auseinandergetrieben (siehe Fig. 9, obere Darstellung für den nicht expandierten Ausgangszustand und die untere Darstellung für einen expandierten Zustand). Je nach Steigung des Spindelgewindes 89 kann der Chirurg durch eine bestimmte Anzahl von Umdrehungen des Handrads 88 eine gewünschte Spreizwirkung erzielen, wodurch das Deckstück 3 höhenmäßig verstellt wird in Bezug auf das Basisstück 2. Zur zusätzlichen Kontrolle des erreichten Expansionszustands ist eine Anzeigeeinrichtung 85 vorgesehen. Sie umfasst eine an den Spreizbacken 81 angeordnete Skalierung 86 sowie einen Indikatorstift 87, welcher an dem Zugorgan 80 angeordnet ist. Der Indikatorstift 87 wird zusammen mit dem Zugorgan bei dem Spreizvorgang nach hinten gezogen, wodurch sich seine Position relativ zur Skala 86 verändert und der somit erreichte Expansionszustand abgelesen werden kann.

Ein zweites alternatives Ausführungsbeispiel für das Zwischenwirbelfusionsimplantat 1' ist in Fig. 11 dargestellt. Es ist im Wesentlichen gleich demjenigen gemäß Fig. 1 aufgebaut, unterscheidet sich jedoch darin, dass es nicht kastenartig gerade sondern im Wesentlichen kreisbogenartig gekrümmt ausgeführt ist. Zu Aufbau und Funktionsweise gilt die vorstehende Beschreibung sinngemäß.

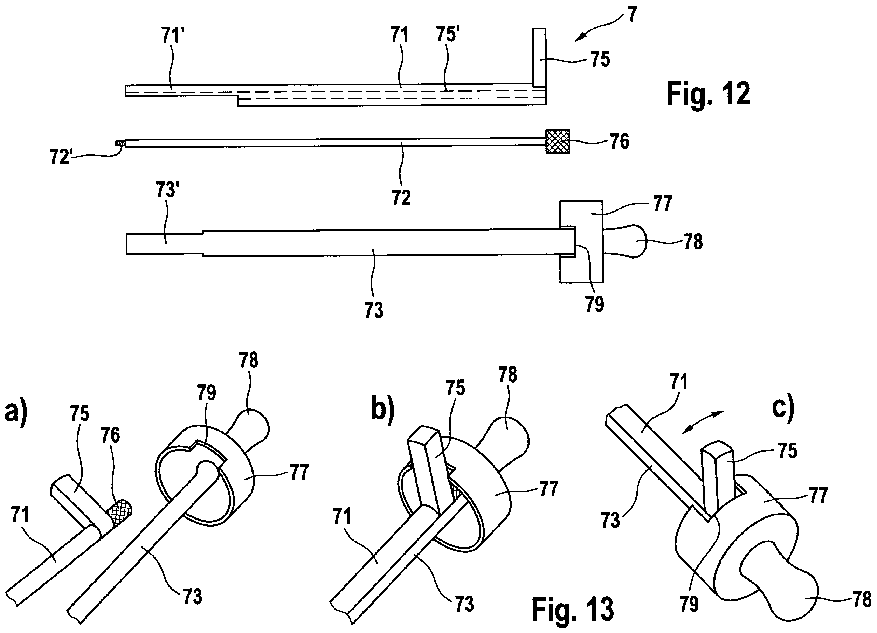

Bei einer weiteren Ausführungsform, welche sich insbesondere für eine transversale Implantation eignet (wie sie in Fig. 1a durch eine gestrichelte Linie in dem Bereich der Bandscheibe 90 angedeutet ist) ist eine weitere Ausführungsform des Zwischenwirbelimplantats vorgesehen. Sie weist im Bereich ihrer Stirnseite 30 eine Öffnung auf, in welcher ein Teil des Betätigungsinstruments eingeschraubt werden kann (siehe gestrichelt dargestellte Öffnung 38 in Fig. 2a). Das dazu vorgesehene Betätigungsinstrument 7 ist in Fig. 12 und 13 dargestellt. Das Bestätigungsinstrument umfasst einen Hebelteil 71 sowie ein Führteil 73. Ferner ist eine Gewindestange 72 vorgesehen, welche in eine entlang der Längsachse des Hebelteils 71 angeordnete Axialbohrung 75' einzusetzen ist. Das Hebelteil 71 weist an seinem hinteren Ende einen radial zur Seite abstehenden Knebel 75 auf. An seinem vorderen Ende weist das Hebelteil 71 eine Greifbacke 71' auf, die zum Ergreifen der Bodenwand 22 des Basisstücks 2 ausgebildet ist. Das Führstück 73 weist an seinem vorderen Ende eine zweite Greifbacke 73', welche in entsprechender Weise zum Ergreifen der Deckwand 33 des Deckstücks 3 ausgebildet ist. Am hinteren Ende weist das Führstück 73 eine Führungsglocke 77 auf, die nach vorne einseitig offen ist und an ihrem hinteren, geschlossenen Ende einen zentralen Handgriff 78 aufweist. An ihrem vorderen, das offene Ende der Glocke umgebenden Rand weist sie eine Ausnehmung 79 auf. Die Ausnehmung 79 ist so bemessen, dass sie in Radialrichtung gesehen eine größere Weite aufweist als der Knebel 75 des Hebelstücks 71.

Zur Montage wird die Gewindestange 72 durch die Axialbohrung 75' des Hebelstücks 71 gesteckt und mit einem Gewinde 72' an ihrem vorderen Ende in ein Schraubgewinde der Öffnung 38 an der Stirnseite 30 des Deckstücks 3 befestigt. Zum Festschrauben dient ein Rändelkopf 76 am hinteren Ende der Gewindestange 72. Das Hebelteil 71 mit dem daran befestigten Deckstück 3 wird sodann an das Führstück 73 angesetzt, und zwar derart, dass es mit seinem Knebel 75 in die Ausnehmung 79 eingreift. Auf das Betätigungsstück 73 wird an dessen vorderen Ende das Basisstück 2 aufgesteckt. Damit ist das Implantat aufgesteckt auf das Instrument 7 in seiner Montageposition, d. h. nicht expandiert.

Die Expansion erfolgt in der Weise, indem das Deckstück 3 relativ zum Basisstück 2 sukzessive verkantet wird, so dass immer auf einer Seite die Rastfeder 42 einen Rastzahn 43 weiter springt. Dieses wechselseitige Verkippen erfolgt in der Weise, indem der Knebel 75 in der Ausnehmung 79 von einer radialen Endposition in die andere radiale Endposition hin und her geschwenkt wird, in Fig. 13c durch den Doppelpfeil dargestellt. Dadurch wird das Deckstück 3 sukzessive verkippt und dabei gleichzeitig expandiert. Für die weitere Beschreibung gilt obige Beschreibung entsprechend.

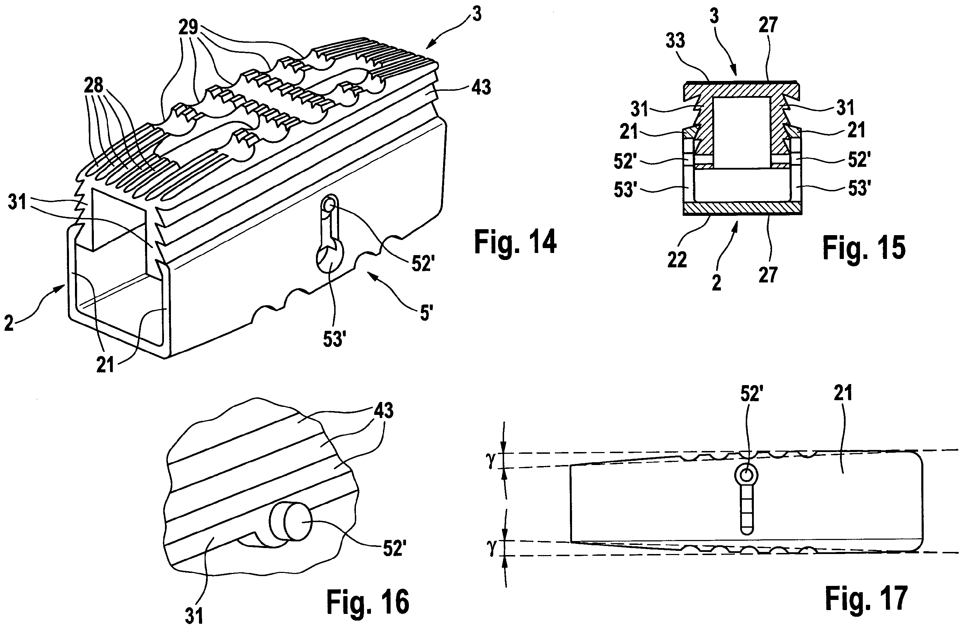

Eine weitere alternative Ausführungsform für das Zwischenwirbelfusionsimplantat ist in Fig. 14 bis 17 dargestellt. Es basiert auf der in Fig. 2 dargestellten Ausführungsform, und gleichartige Komponenten sind mit denselben Bezugsziffern versehen, so dass insoweit auf eine Erläuterung verzichtet wird. Diese Ausführungsform weist eine alternative Fangeinrichtung 5' auf. Sie umfasst einen Querstift 52', der nach außen vorragend unten an den Seitenwänden 31 angeordnet ist (s. auch die Detaildarstellung in Fig. 16). Der Querstift 52' greift in einen fensterartigen Ausschnitt 53', der in den Stehwänden 21 ausgeformt ist. Durch ihre in Vertikalrichtung ausgedehnte Form erlauben sie eine Höhenverstellung des Deckstücks 2, und zwar soweit, bis schließlich der Querstift 52' an das obere Ende des fensterartigen Ausschnitts 53' anschlägt. Damit wird eine weitere Bewegung verhindert. Die Anbringung des Querstifts 52' unten an den Seitenwänden 31 hat den Vorteil, dass die Zahnung 43 an den Seitenwänden ununterbrochen durchlaufen kann. Sie ist damit steifer und kräftiger als die unterbrochene bei der in Fig. 5 dargestellte alternative Ausführung.

Die in Fig. 14 dargestellte Ausführungsform ist ferner mit einer Strukturierung versehen. Sie weist an ihrer Oberseite 33 wie auch an ihrer Unterseite 22 Auskehlungen 29 auf, die in Querrichtung von einer Seite zur anderen verlaufen. Sie sind etwa 2 mm weit und dienen zur besseren Verankerung des Zwischenwirbelfusionsimplantats an den Endplatten 92, 93 der Wirbel 9. Der später im anterioren bzw. posterioren Bereich zum Liegen kommende randnahe Bereich der Oberseite 33 und der Unterseite 22 bleibt frei von den Auskehlungen 29, um die Lasttragfähigkeit in diesen Bereichen nicht zu beeinträchtigen. In Längsrichtung sind eine Vielzahl feiner Rillen 28 ausgebildet, die von einer Stirnseite zur anderen laufen. Sie weisen einen Abstand von etwa 1 mm auf und dienen ebenfalls zur besseren Verankerung. Weiter ist die Oberseite 33 wie auch die Unterseite 22 mit einer Beschichtung aus Titanspray 27 versehen. Sie ist feinkörnig und verstopft die Rillen 28 daher nicht. Diese Beschichtung begünstigt ein An- und Einwachsen von Knochenmaterial. Es sei angemerkt, dass die Strukturierung bzw. die Beschichtung nicht nur auf diese Ausführungsform beschränkt ist, sondern genauso auch bei den übrigen Ausführungsformen vorgesehen sein kann.

Wie aus Fig. 17 zu erkennen ist, kann das Zwischenwirbelfusionsimplantat eine Keilform mit einem Keilwinkel γ aufweisen. Es ist damit besser geeignet zur Implantation zwischen solchen Wirbeln, die in einem stärker gekrümmten Abschnitt der Wirbelsäule angeordnet sind. Es sei angemerkt, dass die Keilform nicht nur auf diese Ausführungsform beschränkt ist, sondern genauso auch bei den übrigen Ausführungsformen vorgesehen sein kann.

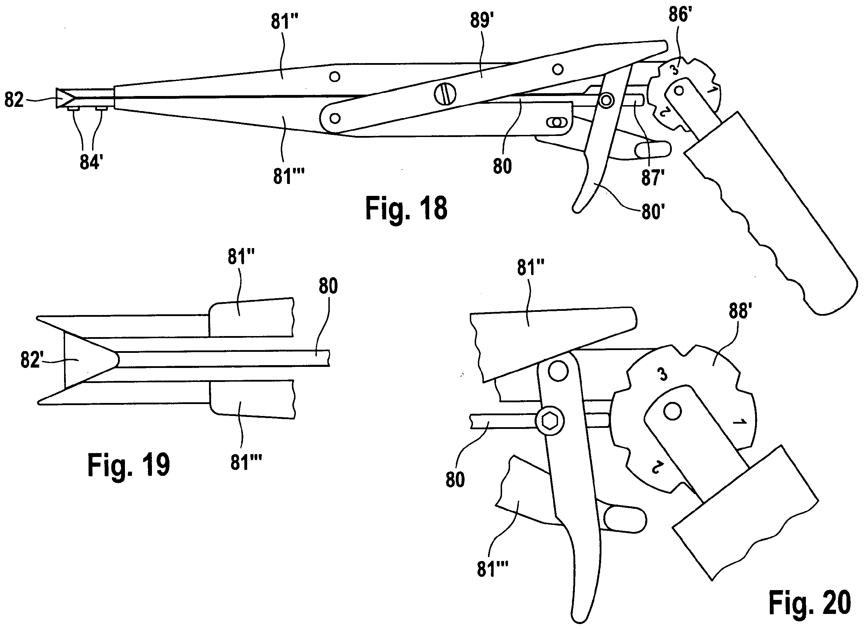

Eine weitere Ausführungsform für ein Betätigungsinstrument 8' ist in Fig. 18 bis 20 dargestellt. Es stimmt mit seinem vorderen, für die Aufnahme des Zwischenwirbelfusionsimplantats ausgebildeten Bereich weitgehend mit dem in Fig. 10 dargestellten Instrument überein. Gleichartige Komponenten tragen gleiche Bezugsziffern, so dass insoweit auf eine Erläuterung verzichtet wird. Das Instrument 8' ist als ein Parallelspreizer ausgeführt. Er wird wie eine Zange betätigt, wobei die Spreizbacken 81", 81"' geradlinig voneinander weg bewegt werden. Als Besonderheit weist das Instrument 8' zum einen einen Kreuzhebel 89' auf, mit dem eine Umkehrkinematik gebildet ist. Sie funktioniert in der Weise, dass beim Zusammendrücken des Instruments 9' im hinteren Griffbereich sich die Spreizbacken 81", 81"' im vorderen Bereich auseinander bewegen. Das vereinfacht die Handhabung beträchtlich. Im Übrigen ist zum Spreizen ein Mechanismus mit einem Ziehkeil 82 vorgesehen, wie auch bei dem in Fig. 9 und 10 dargestellten Instrument.

Zum anderen weist das Betätigungsinstrument 8' eine Anschlageinrichtung 87' auf. Sie beschränkt den Verstellweg des Ziehkeils 82' und damit den Spreizhub. Dazu ist am hinteren Ende des Instruments 8' ein Drehrad 88' vorgesehen, das in vorbestimmte Positionen eingestellt werden kann. Je nach Position bildet es einen mehr oder weniger weit vorragenden Anschlag 87' für das Zugorgan 80, welches über einen Abzugshebel 80' betätigt wird. Der Chirurg braucht nach dem Einsetzen des Implantats zum Spreizen nur den Abzugshebel 80' zu betätigen und zwar soweit, bis das Zugorgan 80 mit seinem hinteren Ende an dem über das Drehrad 88' einstellbaren Anschlag 87 anliegt. Damit ist das Implantat in der gewünschten Weise expandiert. An dem Drehrad sind Markierungen 86' angeordnet, welche die jeweils einstellbaren Expansionsstellungen anzeigen. Durch Einstellen des Handrads 88' auf die gewünschte Expansionsstellung ist automatisch sichergestellt, dass das Betätigungsinstrument 8' beim Erreichen dieser Stellung stoppt. Die Gefahr einer Überexpansion und der daraus resultierenden Risiken für den Patienten ist somit eliminiert.

## Patentansprüche

1. Zwischenwirbelfusionsimplantat zur Fusion zweier benachbarter Wirbel (9, 9') umfassend ein Basisstück (2) und ein Deckstück (3), die jeweils zur Anlage an eine zugewandte Endplatte (92, 93) eines der benachbarten Wirbel (9, 9') ausgebildet sind, wobei das Deckstück (3) höhenverstellbar zum Basisstück (2) ist, eine Ratscheneinrichtung (4) zwischen Basisstück (2) und Deckstück (3) vorgesehen ist, die eine stufige Höhenverstellung bewirkt und eine eingestellte Höhe gegenüber einem Rücklauf sichert,
**dadurch gekennzeichnet, dass**
das Zwischenwirbelfusionsimplantat ferner umfasst eine Fangeinrichtung (5), welche eine Verstellbewegung des Deckstücks (3) begrenzt, und eine Auslöseeinrichtung (6) für die Ratscheneinrichtung (4), um die Höhenverstellung wieder zurückzustellen.

2. Zwischenwirbelfusionsimplantat nach Anspruch 1, **dadurch gekennzeichnet, dass** das Basisstück (2) eine Bodenwand (22) zur Anlage an den einen der benachbarten Wirbel (9) und eine davon aufragende Stehwand (21) umfasst, und das Deckstück (3) eine Deckwand (33) zur Anlage an den anderen der benachbarten Wirbel (9') und eine davon abragende Seitenwand (31), wobei die Stehwand (21) und die Seitenwand (31) Teile der Ratscheneinrichtung (4) sind.

3. Zwischenwirbelfusionsimplantat nach Anspruch 2, **dadurch gekennzeichnet, dass** die Ratscheneinrichtung (4) mehrteilig ist, und eine Mehrzahl von Rastnasen (43) an einem ihrer Teile (31) und eine Rastfeder (42) an einem anderen ihrer Teile (21) aufweist.

4. Zwischenwirbelfusionsimplantat nach Anspruch 3, **dadurch gekennzeichnet, dass** die Rastnasen (43) an der Seitenwand (31) und die Rastfeder (42) an der Stehwand (21) angeordnet sind, oder umgekehrt.

5. Zwischenwirbelfusionsimplantat nach einem der vorangehenden Ansprüche, **dadurch gekennzeichnet, dass** eine doppelte Ratscheneinrichtung (4) vorgesehen ist, vorzugsweise eine an jeder Lateralseite des Implantats (1).

6. Zwischenwirbelfusionsimplantat nach Anspruch 5, **dadurch gekennzeichnet, dass** die beiden Ratscheneinrichtungen (4) höhenmäßig versetzt zueinander sind, vorzugsweise durch einen Versatz ihrer jeweiligen Rastnasen (43).

7. Zwischenwirbelfusionsimplantat nach Anspruch 5 oder 6, **dadurch gekennzeichnet, dass** die Rastnasen (43) an einer der beiden Ratscheneinrichtungen (4) einen Abstand aufweisen, der ein ganzzahliges Vielfaches beträgt - vorzugsweise das Doppelte - des Abstands der Rastnasen (43) an der anderen der beiden Ratscheneinrichtungen (4).

8. Zwischenwirbelfusionsimplantat nach einem der vorangehenden Ansprüche, **dadurch gekennzeichnet, dass** zwei Stehwände (21) seitlich an der Bodenwand (22) vorgesehen sind, und vorzugsweise ausschließlich die Stehwände (21) elastisch sind und die Bodenwand (22) steif ist.

9. Zwischenwirbelfusionsimplantat nach einem der Ansprüche 3 bis 8, **dadurch gekennzeichnet, dass** die Rastnasen (43) asymmetrisch, vorzugsweise sägezahnförmig, ausgebildet sind.

10. Zwischenwirbelfusionsimplantat nach einem der Ansprüche 3 bis 9, **dadurch gekennzeichnet, dass** der Abstand der Rastnasen (43) voneinander etwas des 1,5 bis 3,0-fache ihrer Erhebung beträgt.

11. Zwischenwirbelfusionsimplantat nach einem der Ansprüche 3 bis 10, **dadurch gekennzeichnet, dass** der Winkel β einer tragenden Flanke (45) im Bereich von 5 bis 35° liegt, vorzugsweise von etwa 10°.

12. Zwischenwirbelfusionsimplantat nach Anspruch 11, **dadurch gekennzeichnet, dass** die Rastnasen (43) einander schuppig überlappen, wobei die Gegenflanke (47) vorzugsweise gleichsinnig, aber flacher als die tragende Flanke (43) ausgerichtet ist.

13. Zwischenwirbelfusionsimplantat nach einem der Ansprüche 3 bis 12, **dadurch gekennzeichnet, dass** die Rastfeder (43) formschlüssig in die Rastnasen (42) eingreift, wobei vorzugsweise die Form der Rastnasen (42) so auf die Rastfeder (43) abgestimmt ist, dass eine Selbstsicherung erreicht ist.

14. Zwischenwirbelfusionsimplantat nach Anspruch 13, **dadurch gekennzeichnet, dass** zur Selbstsicherung die ineinander zusammenwirkenden Flächen (45, 46) an Rastnase (43) und Rastfeder (42) nicht parallel sind, sondern spitzwinklig zueinander orientiert sind.

15. Zwischenwirbelfusionsimplantat nach einem der vorangehenden Ansprüche, **dadurch gekennzeichnet, dass** die Fangeinrichtung (5) durch eine Sperrklinke (52) gebildet ist, die mit einem Schultervorsprung (53) zusammenwirkt, und/oder von einem Querstift am Deckstück (2), der in fensterartige Ausschnitte am Basisstück (3) greift.

16. Zwischenwirbelfusionsimplantat nach einem der vorangehenden Ansprüche, **dadurch gekennzeichnet, dass** zwischen Deckstück (3) und Basisstück (2) ein innerer Hohlraum (10) gebildet ist.

17. Zwischenwirbelfusionsimplantat nach Anspruch 16, **dadurch gekennzeichnet, dass** an der Deckplatte (33) und/oder Bodenwand (22) mindestens eine Durchbrechung (34) vorgesehen ist, die mit dem Hohlraum (10) verbunden ist.

18. Zwischenwirbelfusionsimplantat nach Anspruch 16 oder 17, **dadurch gekennzeichnet, dass** ein Sperrelement (70) vorgesehen ist, welches in den Hohlraum (10) einschiebbar ist und das Deckstück (3) in seiner Position sichert.

19. Zwischenwirbelfusionsimplantat nach Anspruch 17 und 18, **dadurch gekennzeichnet, dass** am Sperrelement (70) mindestens eine Zusatzdurchbrechung (74) vorgesehen ist, die mit der Durchbrechung (34) an der Deckplatte (33) und/oder Bodenwand (22) fluchtet.

20. Zwischenwirbelfusionsimplantat nach einem der vorangehenden Ansprüche, **dadurch gekennzeichnet, dass** das Deckstück (3) und/oder Basisstück (2) an seinen Stirnseiten (38) abgerundet ist, insbesondere an seinen Ecken.

21. Zwischenwirbelfusionsimplantat nach einem der vorangehenden Ansprüche, **dadurch gekennzeichnet, dass** das Deckstück und/oder Basisstück an seinen Stirnseiten (38) Öffnungen (30) aufweist.

22. Zwischenwirbelfusionsimplantat nach einem der vorangehenden Ansprüche, **dadurch gekennzeichnet, dass** es kastenförmig gerade oder vorzugsweise kreisbogenartig gekrümmt ausgebildet ist.

23. Zwischenwirbelfusionsimplantat nach einem der vorangehenden Ansprüche, **dadurch gekennzeichnet, dass** am freien Ende der Stehwand (21) und/oder Seitenwand (31) Aufnahmen für ein lateral wirkendes Spreizelement (61) gebildet sind.

24. Zwischenwirbelfusionsimplantat nach Anspruch 23, **dadurch gekennzeichnet, dass** die Aufnahmen Löcher (60) am oberen Ende der Stehwand (21) sind, vorzugsweise im Bereich der Sperrklinke (53).

25. Zwischenwirbelfusionsimplantat nach Anspruch 23 oder 24, **dadurch gekennzeichnet, dass** das Spreizelement ein insbesondere zangenartiges Instrument ist, welches mit seinen Greifern (61) in die Aufnahmen (60) fasst.

26. Zwischenwirbelfusionsimplantat nach einem der vorangehenden Ansprüche, **dadurch gekennzeichnet, dass** das Deckstück (3) aus einem weniger elastischen Material wie das Basisstück (2) besteht.

27. Zwischenwirbelfusionsimplantat nach einem der vorangehenden Ansprüche, **dadurch gekennzeichnet, dass** das Zwischenwirbelfusionsimplantat keilförmig in der Weise ist, dass eine Stirnwand niedriger ist als die andere, und zwar vorzugsweise mit einem Keilwinkel von 3° bis 15°.

28. Zwischenwirbelfusionsimplantat nach einem der vorangehenden Ansprüche, **dadurch gekennzeichnet, dass** die Oberseite (33) des Deckstücks (3) und/oder die Unterseite (22) des Basisstücks (2) strukturiert ausgeführt sind, vorzugsweise in Gestalt von Rillen (29) oder Auskehlungen (28).

29. Zwischenwirbelfusionsimplantat nach Anspruch 28, **dadurch gekennzeichnet, dass** die Ränder der Oberseite (33) und der Unterseite (22) im Bereich von Stirnwänden (30) frei von Strukturierung ist, zumindest aber frei von Auskehlungen (28) .

30. Zwischenwirbelfusionsimplantat nach einem der vorangehenden Ansprüche, **dadurch gekennzeichnet, dass** zumindest die Oberseite (33) und/oder die Unterseite (22) beschichtet sind mit einem Titanplasmaspray (27), das vorzugsweise eine Körnung von höchstens 90 µm aufweist.

31. Anordnung aus einem Zwischenwirbelfusionsimplantat nach einem der Ansprüche 1 bis 30 und einem Betätigungsinstrument für das Zwischenwirbelfusionsimplantat, **dadurch gekennzeichnet, dass** das Betätigungsinstrument (7, 8) einen Querschnitt aufweist, der höchstens so groß wie der des Zwischenwirbelfusionsimplantats (1) ist, das Betätigungsinstrument (8) eine Anzeigeeinrichtung (85) aufweist, welche eine Darstellung proportional zur Höhenverstellung des Zwischenwirbelfusionsimplantats (1) angibt.

## Claims

1. Intervertebral fusion implant for fusion of two adjacent vertebrae (9, 9'), comprising a base piece (2) and a cover piece (3), which are each designed to bear on a facing end plate (92, 93) of one of the adjacent vertebrae (9, 9'), the cover piece (3) being adjustable in height with respect to the base piece (2), a ratchet mechanism (4), provided between base piece (2) and cover piece (3), effecting a stepped adjustment of height and securing an adopted height against reversal, **characterized in that** the intervertebral fusion implant further comprises a catch mechanism (5), which limits an adjustment movement of the cover piece (3), and a release mechanism (6) for the ratchet mechanism (4), in order to reset the height adjustment.

2. Intervertebral fusion implant according to Claim 1, **characterized in that** the base piece (2) has a bottom wall (22), for bearing on one of the adjacent vertebrae (9), and a vertical wall (21) rising from this bottom wall (22), and the cover piece (3) has a cover wall (33), for bearing on the other of the adjacent vertebrae (9'), and a side wall (31) projecting downward from this cover wall (33), said vertical wall (21) and side wall (31) being parts of the ratchet mechanism (4).

3. Intervertebral fusion implant according to Claim 2, **characterized in that** the ratchet mechanism (4) is a multi-part mechanism and has a plurality of locking lugs (43) on one of its parts (31), and a locking spring (42) on another of its parts (21).

4. Intervertebral fusion implant according to Claim 3, **characterized in that** the locking lugs (43) are arranged on the side wall (31) and the locking spring (42) on the vertical wall (21), or vice versa.

5. Intervertebral fusion implant according to one of the preceding claims, **characterized in that** a double ratchet mechanism (4) is provided, preferably one on each lateral face of the implant (1).

6. Intervertebral fusion implant according to Claim 5, **characterized in that** the two ratchet mechanisms (4) are offset in height with respect to each other, preferably with an offset of their respective locking lugs (43).

7. Intervertebral fusion implant according to Claim 5 or 6, **characterized in that** the locking lugs (43) on one of the two ratchet mechanisms (4) have a spacing which is an integral multiple of, preferably twice, the spacing of the locking lugs (43) on the other of the two ratchet mechanisms (4).

8. Intervertebral fusion implant according to one of the preceding claims, **characterized in that** two vertical walls (21) are provided laterally on the bottom wall (22), and preferably only the vertical walls (21) are elastic and the bottom wall (22) is rigid.

9. Intervertebral fusion implant according to one of Claims 3 to 8, **characterized in that** the locking lugs (43) are designed asymmetrically, preferably in a sawtooth shape.

10. Intervertebral fusion implant according to one of Claims 3 to 9, **characterized in that** the spacing of the locking lugs (43) from one another is about 1.5 to 3.0 times their elevation.

11. Intervertebral fusion implant according to one of Claims 3 to 10, **characterized in that** the angle β of a supporting flank (45) is in the range of 5 to 35°, preferably about 10°.

12. Intervertebral fusion implant according to Claim 11, **characterized in that** the locking lugs (43) overlap one another in an imbricated manner, wherein the counter-flank (47) is preferably oriented in the same direction as, but flatter than, the supporting flank (43).

13. Intervertebral fusion implant according to one of Claims 3 to 12, **characterized in that** the locking spring (43) engages with a form fit in the locking lugs (42), the shape of the locking lugs (42) preferably being adapted to the locking spring (43) in such a way that a self-securing effect is obtained.

14. Intervertebral fusion implant according to Claim 13, **characterized in that**, for the self-securing effect, the interacting surfaces (45, 46) on locking lug (43) and locking spring (42) are not parallel but instead oriented at an acute angle to each other.

15. Intervertebral fusion implant according to one of the preceding claims, **characterized in that** the catch mechanism (5) is formed by a pawl (52), which interacts with a shoulder projection (53), and/or by a transverse pin on the cover piece (2), which transverse pin engages in window-like cutouts on the base piece (3).

16. Intervertebral fusion implant according to one of the preceding claims, **characterized in that** an inner hollow space (10) is formed between cover piece (3) and base piece (2).

17. Intervertebral fusion implant according to Claim 16, **characterized in that** at least one aperture (34) is provided on the cover plate (33) and/or bottom wall (22) and is connected to the hollow space (10).

18. Intervertebral fusion implant according to Claim 16 or 17, **characterized in that** a blocking element (70) is provided, which can be pushed into the hollow space (10) and secures the cover piece (3) in its position.

19. Intervertebral fusion implant according to Claim 17 or 18, **characterized in that** at least one additional aperture (74) is provided on the blocking element (70) and is flush with the aperture (34) on the cover plate (33) and/or bottom wall (22).

20. Intervertebral fusion implant according to one of the preceding claims, **characterized in that** the cover piece (3) and/or the base piece (2) is rounded at its end faces (38), in particular at its corners.

21. Intervertebral fusion implant according to one of the preceding claims, **characterized in that** the cover piece and/or base piece has openings (30) on its end faces (38).

22. Intervertebral fusion implant according to one of the preceding claims, **characterized in that** it is designed rectilinearly in a box shape or is preferably curved in the shape of an arc of a circle.

23. Intervertebral fusion implant according to one of the preceding claims, **characterized in that** seats for a laterally acting spreading element (61) are formed at the free end of the vertical wall (21) and/or side wall (31).

24. Intervertebral fusion implant according to Claim 23, **characterized in that** the seats are holes (60) at the upper end of the vertical wall (21), preferably in the area of the pawl (53) .

25. Intervertebral fusion implant according to Claim 23 or 24, **characterized in that** the spreading element is an in particular forceps-like instrument which, with its grippers (61), engages in the seats (60).

26. Intervertebral fusion implant according to one of the preceding claims, **characterized in that** the cover piece (3) is composed of a less elastic material than the base piece (2).

27. Intervertebral fusion implant according to one of the preceding claims, **characterized in that** the intervertebral fusion implant is wedge-shaped, in such a way that one end wall is lower than the other, preferably with a wedge angle of 3° to 15°.

28. Intervertebral fusion implant according to one of the preceding claims, **characterized in that** the top face (33) of the cover piece (3) and/or the bottom face (22) of the base piece (2) are structured, preferably in the form of grooves (29) or furrows (28).

29. Intervertebral fusion implant according to Claim 28, **characterized in that** the edges of the top face (33) and of the bottom face (22) in the area of end walls (30) are free of structuring, or at least free of furrows (28).

30. Intervertebral fusion implant according to one of the preceding claims, **characterized in that** at least the top face (33) and/or the bottom face (22) are coated with a titanium plasma spray (27), which preferably has a grain size of at most 90 µm.

31. Arrangement consisting of an intervertebral fusion implant according to one of Claims 1 to 30 and an actuation instrument for the intervertebral fusion implant, **characterized in that** the actuation instrument (7, 8) has a cross section which at most is as great as that of the intervertebral fusion implant (1), the actuation instrument (8) has a display mechanism (85), which indicates a presentation proportional to the height adjustment of the intervertebral fusion implant (1).

## Revendications

1. Implant de fusion intervertébrale, pour la fusion de deux vertèbres adjacentes (9, 9'), comprenant une partie de base (2) et une partie de recouvrement (3), qui sont à chaque fois réalisées de manière à s'appliquer contre une plaque d'extrémité (92, 93) tournée vers elles de l'une des vertèbres adjacentes (9, 9'), la partie de recouvrement (3) pouvant être réglée en hauteur par rapport à la partie de base (2), un dispositif à cliquet (4) étant prévu entre la partie de base (2) et la partie de recouvrement (3), lequel provoque un réglage en hauteur par degrés et fixe une hauteur ajustée pour l'empêcher de se dérégler,
**caractérisé en ce que**
l'implant de fusion intervertébrale comprend en outre un dispositif de retenue (5) qui limite un mouvement de réglage de la partie de recouvrement (3) et un dispositif de libération (6) pour le dispositif à cliquet (4), afin de réinitialiser le réglage en hauteur.

2. Implant de fusion intervertébrale selon la revendication 1, **caractérisé en ce que** la partie de base (2) comprend une paroi de fond (22) destinée à s'appliquer contre l'une des vertèbres adjacentes (9) et une paroi dressée (21) saillant depuis celle-ci et la partie de recouvrement (3) comprend une paroi de recouvrement (33) destinée à s'appliquer contre l'autre des vertèbres adjacentes (9') et une paroi latérale (31) saillant depuis celle-ci, la paroi dressée (21) et la paroi latérale (31) faisant partie du dispositif à cliquet (4) .

3. Implant de fusion intervertébrale selon la revendication 2, **caractérisé en ce que** le dispositif à cliquet (4) est réalisé en plusieurs parties, et présente une pluralité d'ergots d'encliquetage (43) au niveau de l'une de ses parties (31) et un ressort d'encliquetage (42) au niveau d'une autre de ses parties (21).

4. Implant de fusion intervertébrale selon la revendication 3, **caractérisé en ce que** les ergots d'encliquetage (43) sont disposés au niveau de la paroi latérale (31) et les ressorts d'encliquetage (42) sont disposées au niveau de la paroi dressée (21), ou inversement.

5. Implant de fusion intervertébrale selon l'une quelconque des revendications précédentes, **caractérisé en ce qu'**il est prévu un double dispositif à cliquet (4), de préférence un de chaque côté latéral de l'implant (1).

6. Implant de fusion intervertébrale selon la revendication 5, **caractérisé en ce que** les deux dispositifs à cliquet (4) sont décalés l'un de l'autre en hauteur, de préférence par un décalage de leurs ergots d'encliquetage respectifs (43).

7. Implant de fusion intervertébrale selon la revendication 5 ou 6, **caractérisé en ce que** les ergots d'encliquetage (43) présentent, au niveau de l'un des deux dispositifs à cliquet (4), un espacement qui correspond à un multiple entier - de préférence le double - de la distance des ergots d'encliquetage (43) sur l'autre des deux dispositifs à cliquet (4) .

8. Implant de fusion intervertébrale selon l'une quelconque des revendications précédentes, **caractérisé en ce que** deux parois dressées (21) sont prévues latéralement à la paroi de fond (22), et de préférence exclusivement les parois dressées (21) sont élastiques et la paroi de fond (22) est rigide.

9. Implant de fusion intervertébrale selon l'une quelconque des revendications 3 à 8, **caractérisé en ce que** les ergots d'encliquetage (43) sont réalisés sous forme asymétrique, de préférence en forme de dents de scie.

10. Implant de fusion intervertébrale selon l'une quelconque des revendications 3 à 9, **caractérisé en ce que** la distance entre les ergots d'encliquetage (43) vaut environ 1,5 à 3,0 fois leur hauteur.

11. Implant de fusion intervertébrale selon l'une quelconque des revendications 3 à 10, **caractérisé en ce que** l'angle β d'un flanc porteur (45) est de l'ordre de 5 à 35°, de préférence d'environ 10°.

12. Implant de fusion intervertébrale selon la revendication 11, **caractérisé en ce que** les ergots d'encliquetage (43) se chevauchent les uns les autres à la manière d'écailles, le flanc antagoniste (47) étant orienté de préférence dans le même sens, mais étant plus plat que le flanc porteur (43).

13. Implant de fusion intervertébrale selon l'une quelconque des revendications 3 à 12, **caractérisé en ce que** le ressort d'encliquetage (43) s'engage par engagement par correspondance de formes dans les ergots d'encliquetage (42), la forme des ergots d'encliquetage (42) étant de préférence adaptée au ressort d'encliquetage (43) de telle sorte que l'on obtienne un auto-blocage.

14. Implant de fusion intervertébrale selon la revendication 13, **caractérisé en ce que** pour l'auto-blocage, les surfaces coopérant l'une dans l'autre (45, 46) au niveau de l'ergot 14 d'encliquetage (43) et du ressort d'encliquetage (42) ne sont pas parallèles, mais sont orientées en formant un angle aigu l'une vers l'autre.

15. Implant de fusion intervertébrale selon l'une quelconque des revendications précédentes, **caractérisé en ce que** le dispositif de retenue (5) est formé par un cliquet d'arrêt (52) qui coopère avec une saillie d'épaulement (53) et/ou par une goupille transversale au niveau de la partie de recouvrement (2), qui vient en prise dans des découpes de type fenêtre au niveau de la partie de base (3).

16. Implant de fusion intervertébrale selon l'une quelconque des revendications précédentes, **caractérisé en ce qu'**entre la partie de recouvrement (3) et la partie de base (2) est formée une cavité interne (10).

17. Implant de fusion intervertébrale selon la revendication 16, **caractérisé en ce qu'**au moins un orifice (34) est prévu au niveau de la plaque de recouvrement (33) et/ou de la paroi de fond (22), lequel est connecté à la cavité (10).

18. Implant de fusion intervertébrale selon la revendication 16 ou 17, **caractérisé en ce qu'**un élément de blocage (70) est prévu, lequel peut être inséré dans la cavité (10) et fixe la partie de recouvrement (3) dans sa position.

19. Implant de fusion intervertébrale selon les revendications 17 et 18, **caractérisé en ce qu'**au moins un orifice supplémentaire (74) est prévu au niveau de l'élément de blocage (70), lequel est aligné avec l'orifice (34) au niveau de la plaque de recouvrement (33) et/ou de la paroi de fond (22) .

20. Implant de fusion intervertébrale selon l'une quelconque des revendications précédentes, **caractérisé en ce que** la partie de recouvrement (3) et/ou la partie de base (2) sont arrondies au niveau de leurs côtés frontaux (38), en particulier au niveau de leurs coins.

21. Implant de fusion intervertébrale selon l'une quelconque des revendications précédentes, **caractérisé en ce que** la partie de recouvrement et/ou la partie de base présentent des ouvertures (30) au niveau de leurs côtés frontaux (38).

22. Implant de fusion intervertébrale selon l'une quelconque des revendications précédentes, **caractérisé en ce qu'**il est réalisé en forme de caisson droit ou de préférence sous forme courbe en arc de cercle.

23. Implant de fusion intervertébrale selon l'une quelconque des revendications précédentes, **caractérisé en ce que** des logements pour un élément d'écartement agissant latéralement (61) sont formés au niveau de l'extrémité libre de la paroi dressée (21) et/ou de la paroi latérale (31).

24. Implant de fusion intervertébrale selon la revendication 23, **caractérisé en ce que** les logements sont des trous (60) au niveau de l'extrémité supérieure de la paroi dressée (21), de préférence dans la région du cliquet d'arrêt (53).

25. Implant de fusion intervertébrale selon la revendication 23 ou 24, **caractérisé en ce que** l'élément d'écartement est notamment un instrument en forme de pince qui s'engage dans les logements (60) avec ses organes de préhension (61).

26. Implant de fusion intervertébrale selon l'une quelconque des revendications précédentes, **caractérisé en ce que** la partie de recouvrement (3) se compose d'un matériau moins élastique que celui de la partie de base (2).

27. Implant de fusion intervertébrale selon l'une quelconque des revendications précédentes, **caractérisé en ce que** l'implant de fusion intervertébrale est réalisé en forme de coin de telle sorte qu'une paroi frontale soit inférieure à l'autre, et ce de préférence avec un angle de coin de 3° à 15°.

28. Implant de fusion intervertébrale selon l'une quelconque des revendications précédentes, **caractérisé en ce que** le côté supérieur (33) de la partie de recouvrement (3) et/ou le côté inférieur (22) de la partie de base (2) sont réalisés de manière structurée, de préférence sous la forme de rainures (29) ou de cannelures (28).

29. Implant de fusion intervertébrale selon la revendication 28, **caractérisé en ce que** les bords du côté supérieur (33) et du côté inférieur (22) dans la région des parois frontales (30) sont exempts de structuration, tout au moins exempts de cannelures (28).

30. Implant de fusion intervertébrale selon l'une quelconque des revendications précédentes, **caractérisé en ce qu'**au moins le côté supérieur (33) et/ou le côté inférieur (22) sont revêtus d'un plasma de titane pulvérisé (27), de préférence présentant une granulométrie de 90 µm maximum.

31. Agencement constitué d'un implant de fusion intervertébrale selon l'une quelconque des revendications 1 à 30 et d'un instrument d'actionnement pour l'implant de fusion intervertébrale, **caractérisé en ce que** l'instrument d'actionnement (7, 8) présente une section transversale qui est au moins au maximum aussi grande que l'implant de fusion intervertébrale (1), l'instrument d'actionnement (8) présente un dispositif d'affichage (85) qui indique une représentation proportionnellement au réglage en hauteur de l'implant de fusion intervertébrale (1).
